**Europäisches Patentamt**

**European Patent Office**

**Office européen des brevets**

(19)

(11) Veröffentlichungsnummer: **0 081 152**
**B1**

(12) **EUROPÄISCHE PATENTSCHRIFT**

(45) Veröffentlichungstag der Patentschrift:
**10.04.85**

(21) Anmeldenummer: **82110896.6**

(22) Anmeldetag: **25.11.82**

(51) Int. Cl.⁴: **C 07 C 53/12**, C 07 C 53/08,
C 07 C 69/16, C 07 C 51/12,
C 07 C 51/56, C 07 C 67/36,
C 07 C 67/37

(54) Verfahren zur Herstellung von Essigsäureanhydrid und/oder Essigsäure und/oder Ethylidendiacetat.

(30) Priorität: **04.12.81 DE 3148006**

(43) Veröffentlichungstag der Anmeldung:
**15.06.83 Patentblatt 83/24**

(45) Bekanntmachung des Hinweises auf die Patenterteilung:
**10.04.85 Patentblatt 85/15**

(84) Benannte Vertragsstaaten:
**AT BE CH DE FR GB IT LI NL**

(56) Entgegenhaltungen:
**DE - A - 1 618 499**
**GB - A - 1 045 050**
**GB - A - 1 326 014**

**CHEMICAL ABSTRACTS, Band 85, Nr. 13, 27. September 1976, Seite 592, Nr. 93872d, Columbus, Ohio, USA**
**CHEMICAL ABSTRACTS, Band 85, Nr. 19, 8. November 1976, Seite 489, Nr. 142677e, Columbus, Ohio, USA**

(73) Patentinhaber: **HOECHST AKTIENGESELLSCHAFT,**
**Postfach 80 03 20, D-6230 Frankfurt am Main 80 (DE)**

(72) Erfinder: **Vogt, Wilhelm, Dr., Bellerstrasse 74,**
**D-5030 Hürth (DE)**
Erfinder: **Glaser, Hermann, Magdalenenweg 16,**
**D-5042 Erftstadt (DE)**
Erfinder: **Koch, Jürgen, Dr., Am Römerkanal 10a,**
**D-5040 Brühl (DE)**

ACTORUM AG

## Beschreibung

Die Erfindung betrifft ein Verfahren zur Herstellung von Essigsäureanhydrid und/oder Essigsäure und/oder Ethylidendiacetat durch Umsetzung von Methylacetat und/oder Dimethylether mit Kohlenmonoxid oder Gemischen von Kohlenmonoxid und Wasserstoff bei Temperaturen von 50 bis 300° C und Reaktionsdrucken von 1 bis 500 bar in Gegenwart eines Katalysatorsystems, z. B. gemäss DE-OS Nrn. 2450965, 2836084, 2939839 oder 2941232.

Dabei wurde als wichtigste Komponente des Katalysatorsystems ein Edelmetall der Gruppe VIII des Periodensystems, vorzugsweise Rhodium, Palladium, Iridium oder Ruthenium oder deren Verbindungen, eingesetzt.

Überraschenderweise gelang es nun, diese sehr teuren Edelmetalle durch Rhenium zu ersetzen.

Im einzelnen ist die Erfindung daher dadurch gekennzeichnet, dass man als Katalysatorsystem Rhenium oder dessen Verbindungen; eine Organohalogenverbindung, Halogen oder Halogenwasserstoff; ggf. weitere carbonylbildende Nichtedelmetalle oder deren Verbindungen; ggf. eine tertiäre oder quaternäre organische Stickstoff-, Phosphor-, Arsen- oder Antimonverbindung; und ggf. Alkaliacetat oder Alkaliverbindungen, die unter Reaktionsbedingungen zu Alkaliacetat umgewandelt werden, einsetzt.

Unter weiteren carbonylbildenden Nichtedelmetallen sind insbesondere Cr, Mo, W, Fe, Co oder Ni zu verstehen.

Das Verfahren der Erfindung ist weiterhin wahlweise und bevorzugt dadurch gekennzeichnet, dass man

a) in das Katalysatorsystem mit Methylhalogenid oder Halogenwasserstoff quaternisierte organische Stickstoff- oder Phosphorverbindungen einsetzt, und

b) Methylacetat oder Dimethylether/Rhenium-(-Verbindung)/Halogen(-Verbindung)/Stickstoff-, Phosphor-, Arsen- oder Antimonverbindung/Alkaliverbindung im Molverhältnis 1:(0,0001-0,1):(0,01-1):(0-1):(0-0,1) einsetzt.

Aus „Chemical Abstracts", 85 (1976), 93872 d und 142677 e, sind Umsetzungen von Methylformiat bzw. Methanol mit Methyljodid in Gegenwart von Kohlenmonoxid und Rheniumkatalysatoren zu Essigsäure und Methylacetat bekannt, welches unter den Reaktionsbedingungen nicht zu Essigsäureanhydrid weiterreagiert, so dass vom Verfahren vorliegender Erfindung abweichende Reaktionsmechanismen vorliegen dürften.

Im Rahmen der Erfindung einsetzbare Rheniumverbindungen sind z. B. Rheniumchlorid, Rheniumoxichlorid oder Dirheniumdecacarbonyl.

Das Halogen kann in elementarer Form als Chlor, Brom, Jod oder als Halogenverbindung, vorzugsweise Methylchlorid, Methylbromid, Methyljodid, HCl, HBr, HI eingesetzt werden. Es kommen aber auch andere Alkylhalogenide und Acylhalogenide in Frage, da die Art des Ausgangsmaterials keine erhebliche Rolle spielt.

Die ggf. eingesetzten tertiären oder quaternären organischen Stickstoff-, Phosphor-, Arsen- oder Antimonverbindungen bilden mit Rhenium Komplexe. Als tertiäre Organostickstoff- oder Organophosphorverbindungen kommen Amine, Phosphine oder Aminophosphine in Frage, vorzugsweise Trialkylamine, N,N-Dialkylanilin, Pyridin, Pyrrolidone, Trialkyl- oder Triarylphosphine, insbesondere N-Methylimidazol, 3-Picolin, 2,4-Lutidin, 3,4-Lutidin, Chinolin, Tributylphosphin, Trioctylphosphin, Trilaurylphosphin oder Triphenylphosphin. Die Organostickstoff- oder Organophosphorverbindungen können jedoch auch quaternisiert mit Methylhalogenid oder Halogenwasserstoff eingesetzt werden, z. B. als N-Methylpyridiniumhalogenid, N,N-Dimethylimidazoliumhalogenid, N-Methyl-3-picoliniumhalogenid, N-Methyl-2,4-lutidiniumhalogenid, N-Methyl-3,4-lutidiniumhalogenid, N-Methylchinoliniumhalogenid, Tributylmethylphosphoniumhalogenid, Trioctylmethylphosphoniumhalogenid, Trilaurylmethylphosphoniumhalogenid, Triphenylmethylphosphoniumhalogenid, wobei unter Halogenid jeweils Chlorid, Bromid oder Jodid zu verstehen sind. Als organische Arsen- und Antimonverbindungen können Arsine und Stibine mit Vorteil verwendet werden.

Als Alkaliverbindungen setzt man bevorzugt die Acetate des Kaliums, Rubidiums oder Cäsiums ein.

Die Zusammensetzung der Eingangsgase kann zwischen 100 Vol.-% CO und 20 Vol.-% CO + 80 Vol.-% $H_2$ schwanken. Bevorzugt sind Volumenverhältnisse von CO:$H_2$ = 90:10 bis 50:50.

Vorzugsweise wird bei Temperaturen von 120 bis 250° C und Reaktionsdrucken von 10 bis 300 bar gearbeitet.

Die Reaktion wird in einem Autoklaven aus korrosionsfestem Material, z. B. Edelstahl (Hastelloy) oder emailliertem Stahl, durchgeführt und benötigt eine Verweilzeit von 1 bis 10 h, doch können auch noch kürzere Reaktionszeiten eingestellt werden, so dass die Umsetzung kontinuierlich in einem Strömungsreaktor durchgeführt werden kann.

*Beispiel 1:*

In einen korrosionsfesten Edelstahlrührautoklaven von 1 l Inhalt wurden 250 g Methylacetat, 50 g Methyljodid, 60 g N,N-Dimethylimidazoliumjodid und 2 g Dirheniumdecacarbonyl, $Re_2(CO)_{10}$, eingefüllt. Nach dem Aufdrücken von 80 bar CO und 20 bar Wasserstoff wurde der Autoklav auf 200° C hochgeheizt. Der sich einstellende Maximaldruck betrug 150 bar. Nach einer Verweilzeit von etwa 100 min unter Reaktionsbedingungen fiel der Druck im Autoklaven bis auf 70 bar ab. Nach Aufarbeitung durch Destillation wurden 126,6 g Essigsäureanhydrid, 32,5 g Ethylidendiacetat und 54,2 g Essigsäure erhalten.

*Beispiel 2:*

In den Autoklaven wurde ein Gemisch aus 250 g Methylacetat, 50 g N-Methylimidazol, 50 g Methyljodid, 50 g Methylchlorid und 1,5 g $Re_2(CO)_{10}$ eingesetzt. Nach Aufdrücken von 90 bar CO und 20 bar $H_2$ und Einstellen der Reaktionstemperatur auf 210° C bildete sich im Autoklaven ein Druck von 175 bar aus. Nach 1 h Reaktionszeit war der Druck bis auf 70 bar abgefallen. Das Reaktionsgemisch wurde destillativ aufgearbeitet. Es konnten neben unverändertem Methyljodid und nichtumgesetztem Methylacetat 135 g Essigsäureanhydrid, 28 g Ethylidendiacetat und 32 g Essigsäure erhalten werden.

*Beispiel 3:*

Der nach der destillativen Aufarbeitung in Beispiel 2 angefallene Rückstand wurde nach Zugabe von 250 g Methylacetat und 50 g Methyljodid erneut als Katalysator eingesetzt. CO-Druck: 80 bar, $H_2$-Druck: 20 bar. Bei einer Temperatur von 200°C fiel der Autoklavdruck innerhalb von 30 min von 150 auf 65 bar ab. Anschliessend wurde CO bei 130 bar nachgedrückt, bis keine weitere CO-Aufnahme mehr stattfand. Nach insgesamt 100 min war die Reaktion beendet. Es wurden durch destillative Aufarbeitung erhalten: 290 g Essigsäureanhydrid, 5,5 g Ethylidendiacetat und 40 g Essigsäure neben unverändertem Methyljodid. Methylacetat war nur noch in Spuren nachweisbar.

*Beispiel 4:*

Eingesetzte Mengen: 250 g Methylacetat
50 g Methyljodid
1,5 g $Re_2(CO)_{10}$

Nach dem Spülen des Autoklaven mit Argon wurden 20 bar $H_2$ und 80 bar CO aufgedrückt. Bei einer Temperatur von 200° C war der Druck im Autoklaven innerhalb von 2 h von 160 auf 70 bar abgefallen. Es wurden erhalten: 118 g Essigsäureanhydrid, 23 g Ethylidendiacetat und 12 g Essigsäure.

*Beispiel 5:*

Einsatzmengen: 250 g Methylacetat
50 g Methyljodid
5 g Cäsiumacetat
1,5 g $Re_2(CO)_{10}$

CO-Druck: 80 bar, $H_2$-Druck: 25 bar bei 20° C. Die Reaktionstemperatur wurde auf 205° C eingestellt. Nach einer Reaktionszeit von 2½ h fiel der Autoklavdruck von maximal 160 bar unter Reaktionsbedingungen auf 60 bar ab. Durch destillative Trennung konnten erhalten werden, neben nicht umgesetztem Methylacetat: 102 g Essigsäureanhydrid, 25 g Essigsäure und 28,8 g Ethylidendiacetat.

*Beispiel 6:*

Einsatzmengen: 250 g Methylacetat
50 g Methyljodid
1,5 g $Re_2(CO)_{10}$
10 g Methyltributylphosphoniumjodid

Bei 20° C wurden 80 bar CO und 20 bar $H_2$ aufgedrückt. Bei einer Reaktionstemperatur von 205° C stieg der Autoklavdruck bis auf 180 bar an. Nach 1½ h war die Reaktion beendet. Es konnten aus dem Reaktionsprodukt erhalten werden: 167,7 g Essigsäureanhydrid, 1,5 g Ethylidendiacetat, 34 g Essigsäure.

*Beispiel 7:*

Der nach der destillativen Aufarbeitung in Beispiel 4 angefallene Rückstand wurde aus dem Destillationskolben mit Königswasser aufgenommen und bis zur Trockene eingeengt. Dieser Rückstand wurde nach Zugabe von 250 g Methylacetat, 50 g Methyljodid und 10 g N,N-Dimethylimidazoliumjodid als Katalysator eingesetzt. CO-Druck bei 20° C: 85 bar, $H_2$-Druck bei 20° C: 20 bar. Bei einer Reaktionstemperatur von 210° C fiel der Reaktionsdruck im Autoklaven innerhalb von 110 min auf 70 bar ab. Erhalten wurden 197,7 Essigsäureanhydrid, 17 g Ethylidendiacetat und 33 g Essigsäure.

*Beispiel 8:*

Der nach der destillativen Aufarbeitung in Beispiel 5 angefallene Rückstand wurde wie in Beispiel 7 aufgearbeitet. Nach Zugabe von 250 g Methylacetat, 50 g Methyljodid und 10 g N,N-Dimethylimidazoliumjodid wurden (ohne Wasserstoff) 100 bar CO aufgedrückt und auf 210° C erhitzt. Dabei stieg der Autoklavdruck bis auf 185 bar an. Nach 3½ h war der Druck bis auf 70 bar abgefallen. Durch destillative Aufarbeitung konnten 246,7 g Essigsäureanhydrid erhalten werden. Gleichzeitig waren 20 g Essigsäure entstanden.

*Beispiel 9:*

In den Autoklaven wurden 250 g Methylacetat, 100 g Methylbromid, 30 g N-Methylimidazol und 1,5 g $Re_2(CO)_{10}$ eingefüllt. Nach dem Spülen des Autoklaven mit Argon wurden 80 bar CO und 20 bar $H_2$ aufgedrückt. Bei einer Temperatur von 215° C fiel der Druck innerhalb von 6 h von 180 bar auf 90 bar ab. Nach Abkühlung von destillativer Aufarbeitung wurden 70 g Essigsäureanhydrid und 46 g Essigsäure gewonnen. Ethylidendiacetat war während dieses Versuches nicht entstanden.

*Beispiel 10:*

Einfüllmengen in den Autoklaven: 250 g Methylacetat, 50 g Methyljodid, 60 g N,N-Dimethylimidazoliumjodid, 1,5 g $Re_2(CO)_{10}$, 1 g $I_2$, 80 bar CO, 20 bar $H_2$. Nach Hochheizen des Autoklaven auf 220° C fiel der Druck innerhalb von 130 min von 165 auf 70 bar ab. Das Reaktionsprodukt enthielt nach der destillativen Aufarbeitung 122 g Essigsäureanhydrid, 48 g Essigsäure und 12 g Ethylidendiacetat.

*Beispiel 11:*

Bei einem Einsatz von 250 g Methylacetat, 50 g Methyljodid, 60 g N,N-Dimethylimidazoliumjodid, 1,7 g $ReCl_5$ und 0,87 g Rheniummetall, 80 bar CO, 20 bar $H_2$ in den Autoklaven entstanden bei 185° C nach 8 h Reaktionszeit 135 g Essigsäureanhydrid und 20 g Essigsäure. Die Bildung von Ethylidendiacetat konnte nicht festgestellt werden.

*Beispiel 12:*

Auf ein im Autoklaven befindliches Gemisch aus 250 g Methylacetat, 50 g Methyljodid, 60 g Methyltributylphosphoniumjodid und 1,5 g Re₂(CO)₁₀ wurden 80 bar CO und 20 bar H₂ aufgedrückt. Bei einer Reaktionstemperatur von 215 bis 220° C bildeten sich innerhalb von 5 h 155 g Essigsäureanhydrid, 23 g Essigsäure und 6 g Ethylidendiacetat.

## Patentansprüche

1. Verfahren zur Herstellung von Essigsäureanhydrid und/oder Essigsäure und/oder Ethylidendiacetat durch Umsetzung von Methylacetat und/oder Dimethylether mit Kohlenmonoxid oder Gemischen von Kohlenmonoxid und Wasserstoff bei Temperaturen von 50 bis 300° C und Reaktionsdrucken von 1 bis 500 bar in Gegenwart eines Katalysatorsystems, dadurch gekennzeichnet, dass man als Katalysatorsystem Rhenium oder dessen Verbindungen; eine Organohalogenverbindung, Halogen oder Halogenwasserstoff; ggf. weitere carbonylbildende Nichtedelmetalle oder deren Verbindungen; ggf. eine tertiäre oder quaternäre organische Stickstoff-, Phosphor-, Arsen- oder Antimonverbindung; und ggf. Alkaliacetat oder Alkaliverbindungen, die unter Reaktionsbedingungen zu Alkaliacetat umgewandelt werden, einsetzt.

2. Verfahren nach Anspruch 1, dadurch gekennzeichnet, dass man in das Katalysatorsystem mit Methylhalogenid oder Halogenwasserstoff quaternisierte organische Stickstoff- oder Phosphorverbindungen einsetzt.

3. Verfahren nach einem der Ansprüche 1 oder 2, dadurch gekennzeichnet, dass man Methylacetat oder Dimethylether/Rhenium(-Verbindung)/Halogen(-Verbindung)/Stickstoff-. Phosphor-, Arsen- oder Antimonverbindung/Alkaliverbindung im Molverhältnis 1:(0,0001-0,1):(0,01-1):(0-1):(0-0,1) einsetzt.

## Claims

1. Process for making acetic anhydride and/or acetic acid and/or ethylidene diacetate by reacting methyl acetate and/or dimethylether with carbon monoxide or mixtures of carbon monoxide and hydrogen at temperatures of 50 to 300° C and under reaction pressures of 1 to 500 bar in the presence of a catalyst system, which comprises: using, as the catalyst system, rhenium or its compounds; an organohalogen compound, halogen or hydrogen halide; further carbonyl-yielding non-noble metals or their compounds, if desired; a tertiary or quaternary organic nitrogen, phosphorus, arsenic or antimony compound, if desired; and alkali metal acetate or alkali metal compounds which undergo conversion to alkali metal acetate under the reaction conditions, if desired.

2. Process as claimed in Claim 1, wherein organic nitrogen or phosphorus compounds quaternized with methyl halide or hydrogen halide are used in the catalyst system.

3. Process as claimed in Claim 1 or 2, wherein methyl acetate or dimethylether/rhenium(-compound)/halogen(-compound)/nitrogen, phosphorus, arsenic or antimony compound/alkali metal compound are used in the molar ratio of 1:(0.0001-0.1):(0.01-1):(0-1):(0-0.1).

## Revendications

1. Procédé de préparation d'anhydride acétique et/ou d'acide acétique et/ou de diacétate d'éthylidène par réaction d'acétate de méthyle et/ou d'éther diméthylique avec le monoxyde de carbone ou des mélanges de monoxyde de carbone et d'hydrogène à des températures de 50 à 300° C et sous des pressions de réaction de 1 à 500 bar en présence d'un système catalytique, caractérisé en ce que l'on utilise comme système catalytique le rhénium ou ses composés; un composé organohalogéné, un halogène ou un hydracide halogéné; éventuellement des métaux non nobles supplémentaires formant des carbonyles ou leurs composés; éventuellement un composé organique d'azote, de phosphore, d'arsenic ou d'antimoine tertiaire ou quaternaire; et éventuellement un acétate alcalin ou des composés alcalins se transformant en acétates alcalins dans les conditions réactionnelles.

2. Procédé selon la revendication 1, caractérisé en ce que l'on utilise, dans le système catalytique, un composé d'azote ou de phosphore quaternisé par un halogénure de méthyle ou un hydracide halogéné.

3. Procédé selon l'une des revendications 1 ou 2, caractérisé en ce que l'on utilise l'acétate de méthyle ou l'éther diméthylique, le (composé de) rhénium, le (composé d') halogène, le composé d'azote, de phosphore, d'arsenic ou d'antimoine et le composé alcalin dans des proportions molaires de 1:(0,0001-0,1):(0,01-1):(0-1):(0-0,1).